# EUROPEAN PATENT APPLICATION

(11) **EP 2 463 270 A1**
(43) Date of publication of application: **13.06.2012**
(21) Application number: 11460059.6
(22) Date of filing: 09.12.2011
(51) Int. Cl.: C07C 263/10, C07C 263/20, C07C 265/14

(54) **A method for separating reaction products after toluene diamine (TDA) phosgenation in the gaseous phase during production of toluene diisocyanate (TDI)**

(30) Priority: 10.12.2010 PL 39321510
(71) Applicant: Zaklady Chemiczne ZACHEM S.A., 85-825 Bydgoszcz (PL); POLITECHNIKA WARSZAWSKA, 00-661 Warszawa (PL); Instytut Chemii Przemyslowej im. Prof. Ignacego Moscickiego, 01-793 Warszawa (PL)
(72) Inventor: Slawatycki, Arkadiusz, 85-861 Bydgoszcz (PL); Chrupala, Wojciech, 85-704 Bydgoszcz (PL); Lachmajer, Jerzy, 85-149 Bydgoszcz (PL); Ruczynski, Lech, 85-565 Bydgoszcz (PL); Wójcik, Lucjan, 86-801 Niemcz (PL); Stuczynski, Jacek, 85-801 Bydgoszcz (PL); Baldyga, Jerzy, 05-540 Zalesie Górne (PL); Henczka, Marek, 02-792 Warszawa (PL); Szarlik, Stefan, 05-082 Blizne Laszczynskiego (PL); Lysik, Pawel, 03-982 Warszawa (PL); Sadoska, Teresa, 01-982 Warszawa (PL)
(74) Representative: Twardowska, Aleksandra

(57) **Abstract**

**Summary of the invention**

The invention relates to the method for producing toluene diisocyanate (TDI) by means of toluene diamine (TDA) phosgenation in the gaseous phase and relates to the device for obtaining toluene diisocyanate (TDI) through toluene diamine (TDA) phosgenation in the gaseous phase.

The method for producing toluene diisocyanate (TDI) by means of the toluene diamine (TDA) phosgenation in the gaseous phase, with optional participation of an inert gas, relies on the fact that the main stream of phosgene supply (4) is divided into at least two streams: at least one outer shielding stream, which is fed so as to allow separation of the main area of the reaction from the reactor walls (9), while at least one main inner stream of phosgene (4) is fed to the main area of the reaction.

The device for obtaining toluene diisocyanate (TDI) by means of toluene diamine (TDA) phosgenation in the gaseous phase, with the optional participation of an inert gas, comprises the reactor, the distributor (7) for introducing the raw materials in the gaseous phase to the reactor (9), wherein the distributor (7) is provided with central nozzles for supplying toluene diamine (TDA) and inner nozzles (3) for supplying phosgene, characterised in that the reactor (9), which is shaped like a divergently cylindrical tube, is connected at the narrowest point (8) with the narrowest part of the distributor (7), which is shaped like a convergently cylindrical tube, wherein in the central part of the distributor (7), inner nozzles (3) are located, supplying the main phosgene stream (4), outer annular nozzle (5) that supplies an additional phosgene stream and the main nozzle (2) that supplies toluene diamine (TDA).

## Description

The invention relates to a method for separating reaction products after toluene diamine (TDA) phosgenation in the gaseous phase during production of toluene diisocyanate (TDI).

The subject of the present invention is related to the method of obtaining toluene diisocyanate (TDI) by performing a toluene diamine (TDA) phosgenation reaction in the gaseous phase. It is known that to achieve high efficiency and selectivity of the course of toluene diamine (TDA) phosgenation reaction in the gas phase, rapid mixing of phosgene with TDA is required, as well as avoiding backmixing, as described in a number of patent documents, such as: EP-1319 655, EP-0928785, EP-1275640, EP-0289840, 0749958, EP-1319655, US 2003/0216597, US 2004/0167354, US 2005/0113601, US 2005/0137417, WO 2010/010135. The reaction is subsequently quenched. The process is carried out at a temperature of 300°C to 500°C, and since toluene diisocyanate (TDI) is not thermically stable in such conditions, in order to carry out the process properly, it is required to cool the reaction mixture as quickly as possible, when the reaction with TDA is almost totally completed, to a temperature lower than approximately 150°C, which inhibits TDI decomposition and slows down the precipitation of deposits.

In the known method for inhibiting the reaction and cooling and condensation of reaction products, after TDA and phosgene are reacted to TDI in the gaseous phase, as described in patent documents EP 1403248 and US 2004/0068137, the post-reaction mixture is cooled by coolant injection, most often presented as a stream of liquid dichlorobenzene (DCB), wherein the coolant is sprayed through nozzles, and the inflow of the sprinkling liquid is carried out in a cylindrical extension of a tubular reactor, concurrently in relation to the flowing stream of the reaction mixture that contains products.

In order to increase the velocity of cooling of the hot reaction mixture by using a more advantageous method for cooling of the mixture, therefore a faster quenching of the chemical reactions and condensation of products, another method according to the present invention is provided.

According to the present invention, the method for separating products of toluene diamine (TDA) phosgenation reaction in the gaseous phase during production of toluene diisocyanate (TDI) in a cylindrical reactor for toluene diamine (TDA) phosgenation in the gaseous phase, and condensation of reaction products after phosgenation of (TDA) that contain toluene diisocyanate (TDI) as a main, valuable component, and phosgene, hydrogen chloride and by-products, some of which may form deposits, and, optionally, an inert gas, by spraying the reaction mixture through nozzles located in the walls of the reactor, with a stream of liquid dichlorobenzene (DCB), or another organic cooling agent, being an inert, in the conditions in which the process is carried out, coolant, is based on the fact that the spraying is carried out countercurrently in relation to the stream of flowing reactants.

Preferably, the stream of coolant from the nozzles is directed at an obtuse angle of 135° to 160° in relation to the direction of the post-reaction mixture stream flowing along the axis of the reactor, and the stream opening angle is between 30° to 60°.

It is also preferable that the coolant temperature is between 80°C to 130°C.

It is also advantageous when the nozzles are shielded against contact with liquids that contain ingredients capable of precipitation as deposits, wherein the shields are to be understood as recesses in the part of the reactor wall that serves for cooling or as aerodynamically shaped protruding screens.

Another advantageous feature is to provide no less than two nozzles, preferably two to eight nozzles.

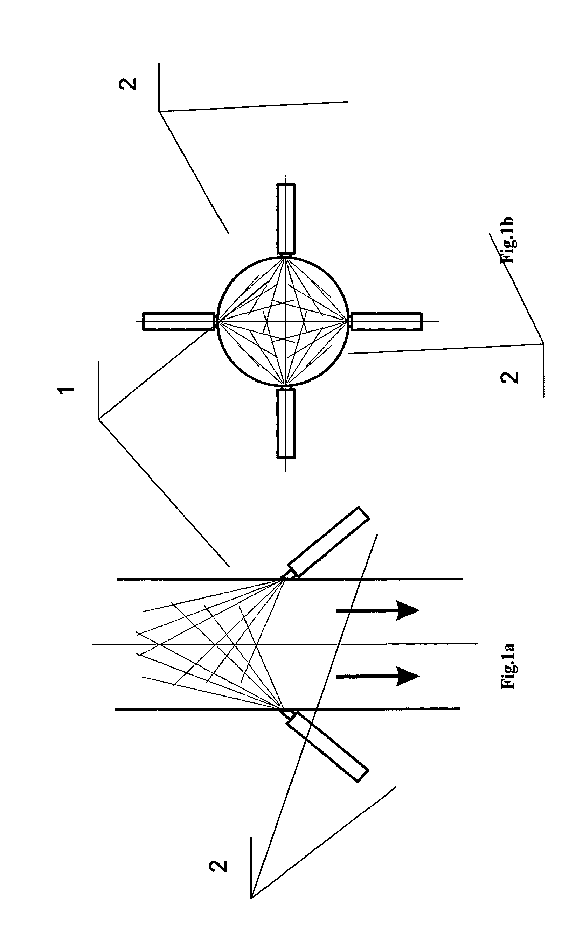

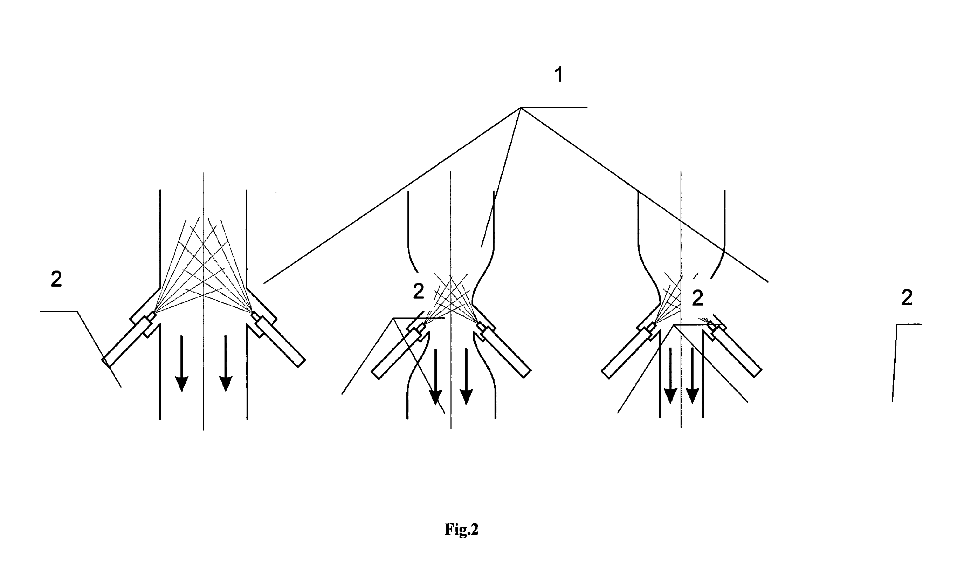

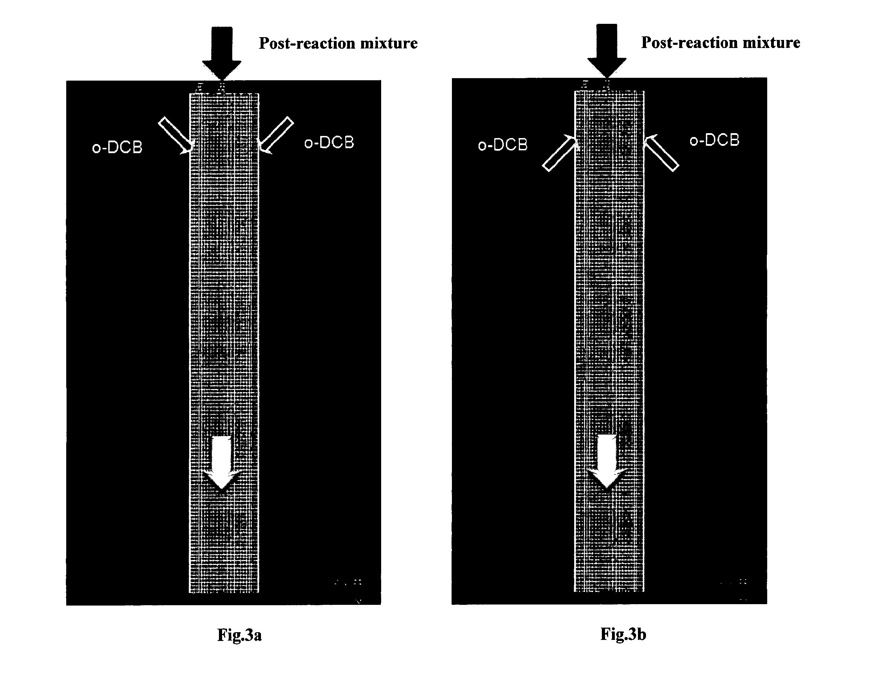

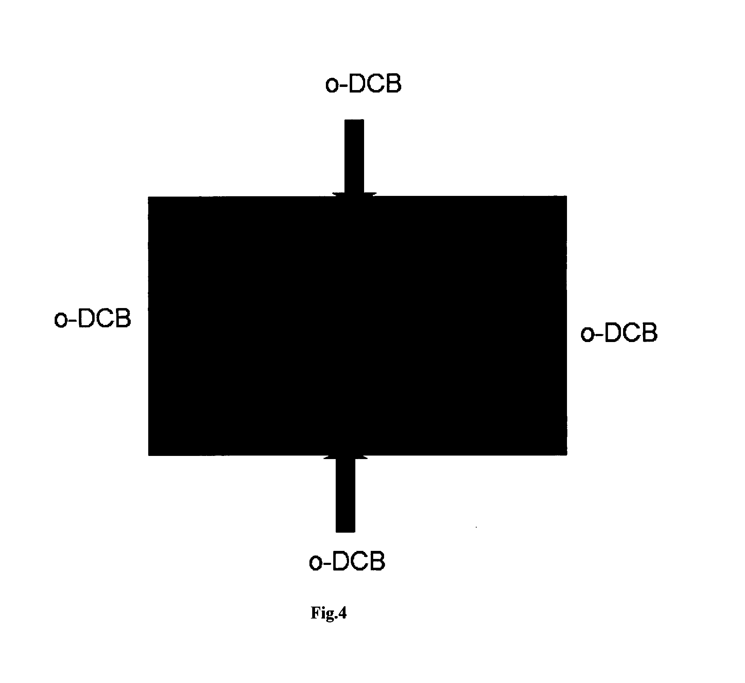

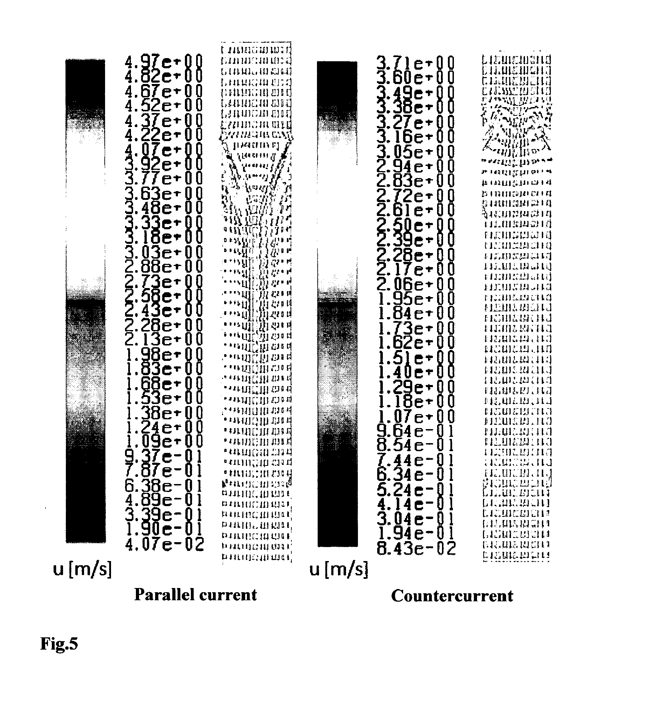

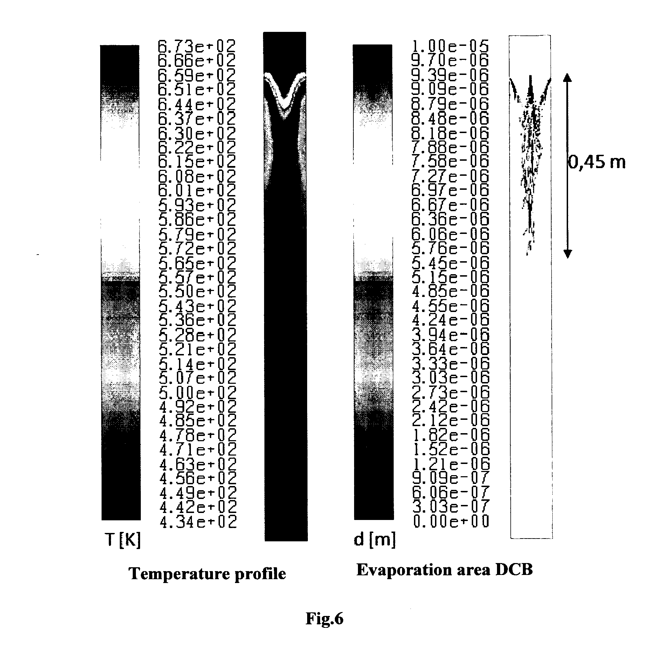

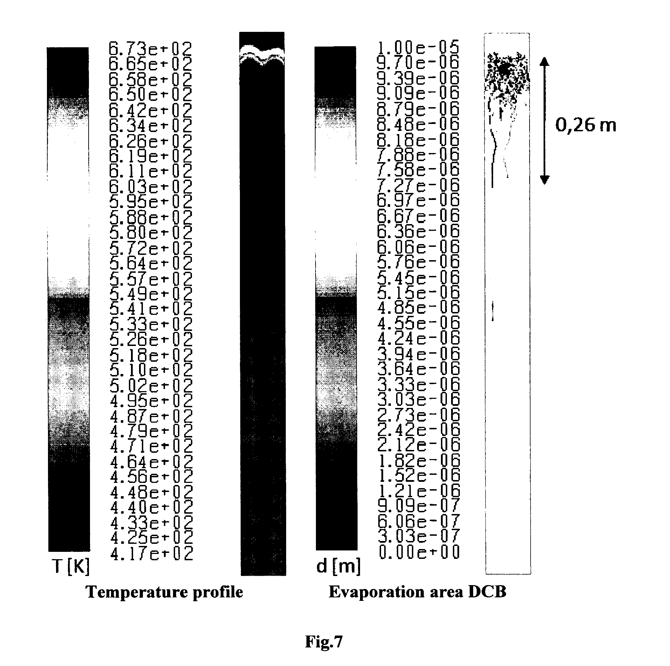

The method of the invention is presented in an embodiment in the drawing, where Fig. 1a shows the section of the reactor along its longitudinal axis with the indicated placement of the nozzles, Fig. 1b shows the section of the reactor transverse to its longitudinal axis with the indicated placement of the nozzles, Fig. 2 shows exemplary variants of shielding of the nozzles in the reactor, Fig. 3a shows a computational grid for model calculations for the course of the cooling process with concurrent flow, Fig. 3b shows a computational grid for model calculations for the course of the cooling process with countercurrent flow, Fig. 4 shows a top view of the computational grid from Fig. 3 a and b, Fig. 5 presents a comparison of the mixture velocity field for concurrent cooling and countercurrent cooling, Fig. 6 shows temperature distribution and the area of coolant evaporation with concurrent cooling, and Fig. 7 shows temperature distribution and the area of coolant evaporation with countercurrent cooling.

As demonstrated in the embodiment in Fig. 1a, spraying nozzles 2 that spray the cooling agent (DBC) or other organic cooling agent, inert under the conditions in which the reaction is carried out, direct the stream of coolant countercurrently in relation to the direction of the reaction products flow. The arrows shown in Fig. 1a present the direction of the reaction products flow. Fig.1b illustrates the arrangement of spraying nozzles 2 in a cross-section of the reactor 1, and Fig. 2 shows an exemplary arrangement of four spraying nozzles 2 at the periphery of reactor 1, wherein the preferred number of nozzles 2 is two to eight.

As shown in Fig. 2, nozzle mouths 2 are protected with shields from the inflow of the post-reaction mixture. In Fig. 2, three examples are shown of how to provide a shield to nozzles 2 through appropriate shaping of the reactor 1 elements, wherein the scope of the invention, as to the possible implementations of the shields, is not limited to the examples presented herein. The shields presented in Fig. 2 are expected to protect the nozzles from contact with liquids containing components capable of precipitation as a deposit, so they must be placed either in the recesses in the part of reactor wall 1, which serves for cooling, or protected by means of aerodynamically shaped screens, which prevent generation of vortices, and thus prevent the formation of backmixing areas. Shields that form a contraction are the most preferred, because they stabilise the flow and increase the speed of the fluid, producing a partial vacuum in the spraying area, which facilitates the distribution of the coolant while hindering its penetration into the walls of reactor 1.

A stream of coolant at a temperature of 80°C to 130°C is directed at an obtuse angle of 135° to 160° flowing along the axis of the reactor 1, and the stream opening angle is between 30° and 60°, as shown in Fig. 1a. The amount of coolant is chosen so that it provides cooling of the reaction mixture to a temperature lower than 130°C, and the use of a countercurrent contributes to the reduction of cooling time by at least 30% compared to the concurrent supply under similar process conditions, wherein the preferred time is shorter than 0.2 seconds.

In order to demonstrate the greater usefulness of the solution that is the subject of the present invention, in comparison with previously patented solutions, exemplary comparative calculations were conducted.

### Comparative Example

In order to determine the extent of the zone where the reaction is inhibited and the coolant is subjected to evaporation, simulation calculations of the course of cooling were performed by means of CFD Fluent 6.3.26 software. Model calculations were performed for a cylindrical spray chamber constituting a reactor with a diameter d = 0.1 m with four spraying nozzles 2, for spraying coolant, symmetrically spaced at every 90°, as shown in Figures 1 and 2. Calculations were performed for two variants of nozzles 2 arrangement for spraying coolant. In the considered variants of the calculations, four nozzles 2 were used, which sprayed coolant in the countercurrent direction, in accordance with the invention, or concurrently to the direction of the gaseous post-reaction mixture flow, according to the prior art. In both cases, the coolant was injected at an angle of 45° relative to the axis of the main stream flow. Calculations were performed in a 3-D system using a computational grid consisting of 172,500 elements. To model the interactions between the continuous phase and the dispersed phase, a standard procedure DPM (Discrete Phase Model) was used.

The main aim of model calculations was to determine the length of the area where the coolant (o-DCB) evaporates and the product (TDI) is subjected to condensation. The length of this zone depends on the initial temperatures of the flowing media, the required temperature drop of the mixture, the coolant flow and the post-reaction gaseous mixture. Fig. 5 shows simulation results of the mixture velocity field in the direct cooling zone for the concurrent and countercurrent supply of coolant to the system. Application of countercurrent cooling causes intensification of mixing in the contact area of both liquids while extending the residence time of the cooled gaseous mixture in the area where the coolant is being injected. As a consequence, this results in an apparent reduction of the area of coolant evaporation and a shortening of the time of reaction inhibition, as shown in Figure 6 for concurrent cooling and in Figure 7 for countercurrent cooling.

The results obtained relate to direct cooling of the post-reaction mixture at a temperature of *Tₚₘ,* with the temperature of the coolant o-DCBO *T_{pc}* = 130°C.

## Claims

1. A method for separating products of toluene diamine (TDA) phosgenation reaction in the gaseous phase during production of toluene diisocyanate (TDI) in a cylindrical reactor for toluene diamine (TDA) phosgenation in the gaseous phase, and condensation of reaction products after phosgenation of (TDA) that contain toluene diisocyanate (TDI) as a main, valuable component, and phosgene, hydrogen chloride and by-products, some of which may form deposits, and, optionally, an inert gas, by spraying the reaction mixture, through nozzles located in the walls of the reactor, with a stream of liquid dichlorobenzene (DCB), or another organic cooling agent, being an inert, in the conditions in which the process is carried out, coolant, **characterised in that** the spraying is carried out countercurrently in relation to the stream of flowing reagents.

2. A method according to claim 1, **characterised in that** the stream of coolant from the nozzles is directed at an obtuse angle of 135° to 160° in relation to the direction of the post-reaction mixture stream flowing along the axis of the reactor, and the stream opening angle is between 30° to 60°.

3. A method according to claim 1 or 2, **characterised in that** the coolant temperature is between 80° and 130°C.

4. A method according to claim 1, **characterised in that** the nozzles 2 are shielded against contact with liquids that contain ingredients capable of precipitation as deposits, wherein the shields are to be understood as recesses in the part of the reactor wall 1 that serves for cooling or as aerodynamically shaped protruding screens.

5. A method according to claim 1, **characterised in that** not less than two nozzles 2 are used.

6. A method according to claim 5, **characterised in that** two to eight nozzles 2 are used.
